# EUROPEAN PATENT SPECIFICATION

(11) **EP 1 522 316 B1**
(45) Date of publication and mention of the grant of the patent: **21.02.2018**
(21) Application number: 03764126.3
(22) Date of filing: 02.07.2003
(51) Int. Cl.: A61K 47/34, A61K 47/10, A61K 47/14, A61K 9/06, A61K 9/08, A61K 9/12, A61K 9/70

(54) **TRANSDERMAL ABSORPTION PREPARATION**
TRANSDERMALES ABSORPTIONSPRÄPARAT
PREPARATION A ABSORPTION TRANSDERMIQUE

(30) Priority: 16.07.2002 JP 2002206565
(43) Date of publication of application: 13.04.2005
(73) Proprietor: Hisamitsu Pharmaceutical Co., Inc., Tosu-shi, Saga 841-0017 (JP)
(72) Inventor: NARUI, Takashi, Sakura-shi, Chiba 285-0817 (JP); OMICHI, Katsuhiro, Saitama-shi, Saitama 338-0832 (JP); OKADA, Minoru, Inzai-shi, Chiba 270-1323 (JP); KURAZUMI, Toshiaki, Narita-shi, Chiba 286-0011 (JP)
(74) Representative: Hartz, Nikolai
(86) International application number: PCT/JP2003/008400
(87) International publication number: WO 2004/006960

(56) References cited:
- EP-A1- 0 097 374
- EP-A1- 1 043 020
- WO-A1-96/33706
- WO-A1-99/01137
- WO-A1-99/33458
- WO-A2-01/76561
- JP-A- 3 197 423
- JP-A- 5 000 934
- JP-A- 5 178 763
- JP-A- 6 239 740
- JP-A- 7 285 861
- JP-A- 10 182 450
- JP-A- 62 238 216
- JP-A- 2001 026 553
- JP-A- 2003 093 152
- US-A- 4 244 942

## Description

### TECHNICAL FIELD

The present invention relates to a novel transdermal absorption preparation and, more particularly, to a transdermal absorption preparation containing a transdermal absorption promotion composition that can promote transdermal absorption of a compound having a comparatively high lipophilic physiological activity (a drug) and a drug.

### BACKGROUND ART

As a method of administering a drug to a patient, per-oral medication, transdermal medication, medication by injection, and the like can be given. When a drug is orally administered to a patient, some drugs may exhibit decreased bioavailability due to a first-pass effect in the liver and other drugs may cause a side reaction in an alimentary canal. In addition, it may be difficult to orally administer a drug to a patient depending on the clinical condition. On the other hand, injection not only may cause pain to the patient, but also requires the patient to visit a medical institution to receive the medication. In addition, handling of an injection instrument is complicated. For these reasons, dermal administration of drugs in place of these methods of administration is attracting attention due to advantages such as simple medication and the like.

However, dermal administration has a basic problem of low permeability of a drug due to a barrier function of the horny layer on the skin surface against absorption of the drug. In particular, drugs having a large molecular weight or having high water solubility are said to exhibit low permeability through the skin.

For these reasons, it is essential for effective dermal medication of drugs to promote dermal absorption of the drugs in some manner. As a method for solving this problem, use of Azone (1-dodecylazacycloheptan-2-on), menthol, pyrrolidone, or terpens independently or in combination of two or more as transdermal penetration enhancers has been studied.

However, these transdermal penetration enhancers do not exhibit a sufficient effect and are not necessarily effective for all drugs. Some transdermal penetration enhancers that exhibit some effect of dermal absorption promotion present a disagreeable odor, are sticky, exhibit an unfavorable feeling of use, and strongly stimulate the skin. Other transdermal penetration enhancers are unstable themselves or impair the stability of a drug.

EP 1 043 020 A1 discloses a medicinical composition for percutaneous administration comprising amidine derivatives as drug component.

US 4,244,942 A discloses a creamy preparation containing a steroid as drug component.

JP 2003 093152 A relates to a cosmetic impregnated sheet. Loperamide hydrochloride or lidocaine as drug component are not mentioned.

JP H10-182450 A discloses a composition for external use comprising diclofenac as drug component.JP H07-285861 A discloses an external preparation comprising benzonitrile derivatives as drug component.

WO 96/33706 A1 discloses compositions for inducing dermal analgesia, comprising an anaestetic agent like lidocaine, solubilising agents like propylene glycol, lipophilic permeation enhancer like propylene glycol monolaurate, and polyoxyethylene ethers. This document does not disclose lauromacrogol as polyoxyethylene ether.

An object of the present invention is to overcome the above problems and provide a transdermal absorption preparation that can not only exhibit an excellent transdermal absorption promotion effect, but also exhibit superior skin-permeability, even if a drug having a relatively high lipophilic property and poor transdermal absorbability is used, exhibit a favorable feeling of use, and are safe and stable.

### DISCLOSURE OF THE INVENTION

The present inventors have conducted extensive studies to achieve the above object and found that a transdermal absorption promotion composition containing propylene glycol, a polyol fatty acid ester, and a lauromacrogol remarkably increases permeability of drugs through the skin. The present inventors have further found that the transdermal absorption preparation prepared by adding a drug to the transdermal absorption promotion composition exhibits remarkably excellent transdermal absorptivity of the drug, a favorable feeling of use, and stability, and imparts only slight stimulation to the skin. These findings have led to the completion of the present invention.

Specifically, the present invention makes use of a transdermal absorption promotion composition comprising the following components (a), (b), and (c):
(a) propylene glycol,
(b) a polyol fatty acid ester, and
(c) lauromacrogol.

The present invention provides a transdermal absorption preparation comprising the following components (a), (b), (c), and (d) :
(a) propylene glycol,
(b) a polyol fatty acid ester,
(c) lauromacrogol, and
(d) loperamide hydrochloride or lidocaine as a drug component.

### BRIEF DESCRIPTION OF THE DRAWINGS

Figure 1 is a drawing showing the relationship between the amounts of accumulated loperamide hydrochloride extracted from solutions of Example 1 and Comparative Examples 1-3 which penetrated the rat skin and the time elapsed.
Figure 2 is a drawing showing the relationship between the accumulated amounts of the drug extracted from ointments of Example 2 and Comparative Examples 4-6 which penetrated the rat skin and the time elapsed.

### BEST MODE FOR CARRYING OUT THE INVENTION

The amount of propylene glycol used in the transdermal absorption promotion composition of the present invention (hereinafter referred to simply as "composition of the present invention") as the component (a) is usually 1-99 wt%, preferably 4-95 wt%, and particularly preferably 10-90 wt% ("wt%" is hereinafter indicated as "%").

The polyol fatty acid ester used as the component (b) in the present invention composition may be an ester of a polyol and a fatty acid. The ester may be either a monoester or a diester. As the polyol forming the polyol fatty acid ester, ethylene glycol, propylene glycol, butylene glycol, glycerol, sorbitan, tetra glycerol, and the like can be given. As the fatty acid, saturated fatty acids such as caprylic acid, capric acid, octanoic acid, isooctanoic acid, lauric acid, myristic acid, palmitic acid, and the like can be given. As specific examples of the polyol fatty acid, ethylene glycol monocaprylate, ethylene glycol monoisooctanate, propylene glycol monocaprylate, propylene glycol dicaprylate, propylene glycol dicaprate, glycerol monocaprylate, solbitan monocaprylate, tetraglycerol monocaprylate, and the like can be given. These are used either individually or in combination of two or more.

The component (b) is preferably a compound with excellent mutual solubility with propylene glycol of the component (a), for example, ethylene glycol monocaprylate, ethylene glycol monoisooctanoate, or the like. Propylene glycol monocaprylate is particularly preferable as the polyol fatty acid ester.

The lauromacrogol of the component (c) is also preferably those having excellent mutual solubility with propylene glycol of the component (a). Although not specifically limited, lauryl ethers with 2-25 mol, preferably 2-9 mol ethylene oxide addition are preferable.

With regard to the ratio of the components (a), (b), and (c) in the composition of the present invention, the ratio of the total weight of the component (b) and component (c) to the weight of the component (a) is 0.01-99, preferably 0.05-25, and more preferably 0.1-9, and the weight ratio of the component (c) to the component (b) is 0.1-10, preferably 0.25-4, and more preferably 0.5-2.

The composition of the present invention can be produced by sufficiently mixing to homogenize the above components (a), (b), and (c) by a conventional method. An excellent transdermal absorption property can be obtained by incorporating a drug component to the resulting composition.

The transdermal absorption preparation of the present invention can be prepared by incorporating loperamide hydrochloride or lidocaine as the drug component (d) together with the above components (a), (b), and (c).

The drug can promote transdermal absorption when used together with the composition used in the present invention, and a drug with comparatively high lipophilic properties is preferable. The drug with comparatively high lipophilic properties indicates the properties of a drug in which a part or all of the drug incorporated in the preparation is dissolved in propylene glycol of the component (a).

Reference examples of such a drug which are not according to component (d) of the preparation of the invention include, but are not limited to, steroid anti-inflammation agents such as prednisolone and hydrocortisone, non-steroid anti-inflammation agents such as indomethacin and diclofenac, as well as their ester derivatives, antihistamines such as a diphenhydramine, central-nerve agonists such as isoprenaline hydrochloride, hormones such as estradiol, hypotensors such as furosemide, cardiotonics such as digitoxin, anti- arrhythmic agents such as disopyramide phosphate, coronary vasodilators such as tolazoline hydrochloride, local anesthetics, painkillers such as acetaminophen, muscle relaxants such as suxamethonium chloride, antifungals such as clotrimazole, anti-cancer drugs such as fluorouracil, urination problem improvers such as tamsulosin hydrochloride, anti-epileptic drugs such as diazepam, anti-Parkinson's disease agents such as bromocriptine mesilate, non-smoking adjuvants such as nicotine, vitamins, and prostaglandins.

The amount of the component (d) to be incorporated into the preparation of the present invention varies according to the type and form of the drug, and the like, but is usually 0.01-10%, preferably 0.05-5%, and more preferably 0.1-3% of the preparation.

The amount of the composition of the present invention to be incorporated when preparing the preparation of the present invention varies according to the type and amount of the drug, the form of the preparation, and the like, but is usually 1-99.99%, preferably 2-99.9%, and more preferably 5-99% of the preparation.

Various forms that can cause the drug to be absorbed by the skin are possible for the preparation of the present invention without any specific limitations. Specific examples of the form include ointment, gel, cream, gel-like cream, liquid, lotion, aerosol, liniment, plaster, poultice, and reservoir-type patch.

The preparation of the present invention can be produced by combining the above components (a) to (d) with other components for forming the preparation such as a vehicle, an adjuvant, and other optional additives.

For example, when preparing an ointment, an ointment base such as petroleum jelly or macrogol, adjuvants such as paraffin, light anhydrous silicic acid, and a surfactant, a stabilizer such as dibutylhydroxytoluene or sodium edentate, and, as required, a pH adjusting agent and the like are added to the above components (a) to (d).

When preparing a gel, a lower alcohol such as ethyl alcohol or isopropyl alcohol, purified water, a gelling agent such as carboxyvinyl polymer or ethylcellulose, a neutralizer such as triethanolamine, and the like are added to the above components (a) to (d). To prepare a cream, a higher fatty acid ester such as myristic acid ester or palmitic acid ester, a hydrocarbon such as liquid paraffin, purified water, an emulsifying agent such as polyoxyethylene alkyl ether, and the like are added to the above components (a) to (d).

To prepare a gel-like cream, a higher fatty acid ester such as myristic acid ester or palmitic acid ester, a hydrocarbon such as liquid paraffin, purified water, an emulsifying agent such as polyoxyethylene alkyl ether, a gelling agent such as carboxyvinyl polymer, a neutralizer such as diisopropanolamine, and the like are added to the above components (a) to (d). To prepare a liquid preparation, a liquid higher fatty acid, a vegetable oil, and the like are added to the above components (a) to (d).

To prepare a lotion, the above components (a) to (d) are dissolved in, emulsified by, or suspended in a lower alcohol such as ethyl alcohol or isopropyl alcohol, or a mixture of the lower alcohol and purified water. To prepare a liniment, the above components (a) to (d) are combined with a lower alcohol, a fatty oil, and the like.

An aerosol agent can be prepared by filling an aerosol container with the above-mentioned liquid preparation, lotion, or liniment, for example, together with an injection agent such as liquefied petroleum gas. A plaster can be prepared by adding the above-mentioned components (a) - (d) to a substrate agent such as an ethylene vinyl acetate agglutinant, a styrene-isoprene-styrene block copolymer, and the like. A poultice can be prepared by adding the above-mentioned components (a) - (d) to an ointment base formed from a partially neutralized polyacrylic acid, sodium polyacrylate, and the like. A reservoir-type patch can be prepared by adding the above-mentioned components (a) - (d) to a drug reservoir layer formed from a lower alcohol, purified water, water soluble polymer, fatty alcohol, paraffin, silicone, and the like.

One preferable embodiment of the preparation of the present invention is a homogeneous solution consisting essentially of the above components (a) to (d). Another preferable embodiment of the preparation of the present invention is an oily ointment or a cream containing the above components (a) to (d) and a oily base component.

These preparations of the present invention can promote transdermal absorption of the drug components contained in the preparations when applied to the skin. The preparations of the present invention can particularly promote transdermal absorption of drugs with relatively high lipophilic properties which have been difficult to be transdermally absorbed in the skin using a conventional absorption preparation.

Although the detailed mechanism of promoting transdermal absorption using the composition of the present invention has still to be elucidated, it can be presumed that the polyol fatty acid ester or lauromacrogol reduce the barrier capability of the skin horny layer, penetrate into the horny layer, and react with the horny layer, causing the drug dissolved in propylene glycol to synergistically react and exhibit the pharmaceutical action.

### EXAMPLES

The present invention will be described in more detail by examples, which should not be construed as limiting the present invention.

### Example 1

Propylene glycol monocaprylate, polyoxyethylene (2) lauryl ether, and propylene glycol were mixed at a weight ratio of 1:1:8 to obtain a transdermal absorption promotion composition. One part by weight of loperamide hydrochloride was dissolved in 99 parts by weight of the transdermal absorption promotion composition to prepare a homogeneous solution.

### Comparative Example 1

One part by weight of loperamide hydrochloride was dissolved in 99 parts by weight of propylene glycol to prepare a homogeneous solution.

### Comparative Example 2

Propylene glycol monocaprylate and propylene glycol were mixed at a weight ratio of 1:9 to obtain a transdermal absorption promotion composition. One part by weight of loperamide hydrochloride was dissolved in 99 parts by weight of the transdermal absorption promotion composition to prepare a homogeneous solution.

### Comparative Example 3

Polyoxyethylene (2) lauryl ether and propylene glycol were mixed at a weight ratio of 1:9 to obtain a transdermal absorption promotion composition. One part by weight of loperamide hydrochloride was dissolved in 99 parts by weight of the transdermal absorption promotion composition to prepare a homogeneous solution.

### Test Example 1

### Skin penetration test (1)

The skin extracted from the back of an HWY/Slc male rat (age: nine weeks) was applied to a Franz-type diffusion cell (application area: 2.83 cm²) in which water at a temperature of 37°C was circulated. 17 ml of a physiological saline solution was put into the receiver (dermis) side and stirred using a magnetic stirrer. Solutions of Example 1 and Comparative Examples 1-3, 28 µl each, were respectively applied to the donor (horny layer) side. The solution in the receiver was extracted over time to determine the concentration of loperamide hydrochloride using high performance liquid chromatography. The amount of the loperamide hydrochloride that penetrated the rat skin was calculated.

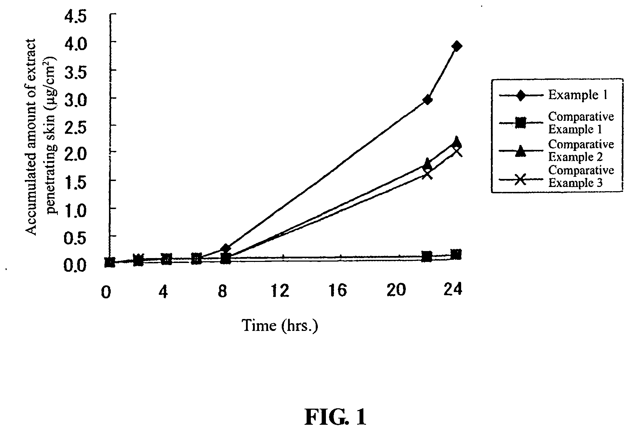

The relationship between the amounts of accumulated loperamide hydrochloride extracted from solutions of Example 1 and Comparative Examples 1-3 that penetrated the rat skin and the time elapsed is shown in Figure 1. As clear from Figure 1, the solution of Example 1 which contains propylene glycol, propylene glycol monocaprylate, and polyoxyethylene (2) lauryl ether was confirmed to exhibit higher skin permeability than the solution of Comparative Example 1 containing only propylene glycol, the solution of Comparative Example 2 containing propylene glycol and propylene glycol monocaprylate, and the solution of Comparative Example 3 containing propylene glycol and polyoxyethylene (2) lauryl ether.

### Example 2

One part by weight of lidocaine was dissolved in 10 parts by weight of propylene glycol. 4 parts by weight of propylene glycol monocaprylate, 4 parts by weight of polyoxyethylene (2) lauryl ether, 5 parts by weight of sorbitan sesquioleate, and 4 parts by weight of paraffin were added to and dissolved in the solution. Then, 1.5 parts by weight of light anhydrous silicate and 70.5 parts by weight of white petroleum jelly were added to prepare an ointment.

### Comparative Example 4

One part by weight of lidocaine was dissolved in 10 parts by weight of propylene glycol. 4 parts by weight of sorbitan sesquioleate and 3 parts by weight of paraffin were added to and dissolved in the solution. Then, 82 parts by weight of white petroleum jelly was added to prepare an ointment.

### Comparative Example 5

One part by weight of lidocaine was dissolved in 10 parts by weight of propylene glycol. 3.5 parts by weight of propylene glycol monocaprylate, 4 parts by weight of sorbitan sesquioleate, and 3 parts by weight of paraffin were added to and dissolved in the solution. Then, 78.5 parts by weight of white petroleum jelly was added to prepare an ointment.

### Comparative Example 6

One part by weight of lidocaine was dissolved in 10 parts by weight of propylene glycol. 4 parts by weight of polyoxyethylene (2) lauryl ether, 4 parts by weight of sorbitan sesquioleate, and 3 parts by weight of paraffin were added to and dissolved in the solution. Then, 78 parts by weight of white petroleum jelly was added to prepare an ointment.

### Test Example 2

### Skin penetration test (2)

The skin extracted from the back of an HWY/Slc male rat (age: nine weeks) was applied to a Franz-type diffusion cell (application area: 2.83 cm²) in which water at a temperature of 37°C was circulated. 17 ml of a physiological saline solution was put into the receiver (dermis) side and stirred using a magnetic stirrer. Ointments of Example 2 and Comparative Examples 4-6, 28 mg each, were respectively applied to the donor (horny layer) side. The solution in the receiver was extracted over time to determine the concentration of lidocaine using high performance liquid chromatography. The amount of the lidocaine that penetrated the rat skin was calculated.

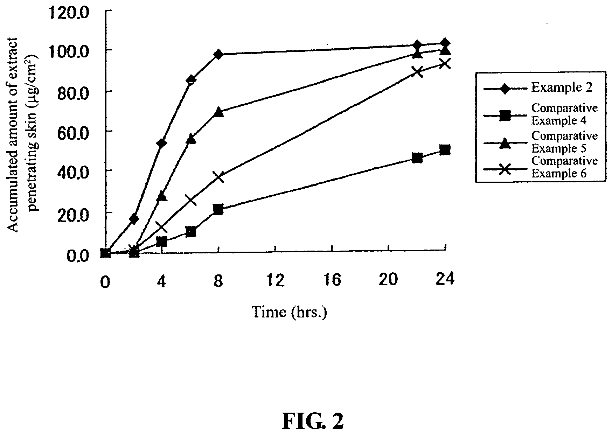

The relationship between the amounts of accumulated drug that penetrated the rat skin from the ointments of Example 2 and Comparative Examples 4-6 and the time elapsed is shown in Figure 2. As clear from Figure 2, the solution of Example 2 which contains propylene glycol, propylene glycol monocaprylate, and polyoxyethylene (2) lauryl ether was confirmed to exhibit higher skin permeability than the solution of Comparative Example 4 containing only propylene glycol, the solution of Comparative Example 5 containing propylene glycol and propylene glycol monocaprylate, and the solution of Comparative Example 6 containing propylene glycol and polyoxyethylene (2) lauryl ether.

### INDUSTRIAL APPLICABILITY

The transdermal absorption preparation containing the composition and a drug component not only exhibit a transdermal absorption promotion effect, but also exhibit superior skin-permeability, even if a drug having a relatively high lipophilic property and poor transdermal absorbability is used, exhibit a favorable feeling of use, and are safe and stable.

Therefore, the transdermal absorption preparation containing the composition and a drug component are useful for transdermally administering various drugs to patients.

## Claims

1. A transdermal absorption preparation comprising the following components (a), (b), (c), and (d):
(a) propylene glycol,
(b) a polyol fatty acid ester,
(c) lauromacrogol, and
(d) loperamide hydrochloride or lidocaine as a drug component.

2. The transdermal absorption preparation according to claim 1, wherein the preparation is in the form of an ointment, gel, cream, gel-like cream, liquid, lotion, aerosol, liniment, plaster, poultice, or reservoir-type patch.

3. The transdermal absorption preparation according to claim 1, wherein the preparation is a homogeneous liquid preparation basically comprising the components (a) to (d).

4. The transdermal absorption preparation according to claim 1, wherein the preparation is an oily ointment or oily cream preparation comprising the components (a) to (d) and an oily base.

5. The transdermal absorption preparation according to claim 1, wherein the components (b) and (c) are mutually soluble with the component (a).

6. The transdermal absorption preparation according to claim 1, wherein the ratio of the total weight of the component (b) and component (c) to the weight of the component (a) is 0.01-99 and the weight ratio of the component (c) to the component (b) is 0.1-10.

## Patentansprüche

1. Ein transdermales Absorptionspräparat umfassend die folgenden Komponenten (a), (b), (c), und (d):
(a) Propylenglycol,
(b) einen Polyolfettsäureester,
(c) Lauromacrogol und
(d) Loperamid-Hydrochlorid oder Lidocain als Wirkstoffkomponente.

2. Das transdermale Absorptionspräparat gemäß Anspruch 1, wobei das Präparat in Form einer Salbe, eines Gels, einer Creme, einer gelartigen Creme, einer Flüssigkeit, einer Lotion, eines Aerosols, eines Einreibemittels, eines Pflasters, eines Umschlags oder eines Reservoirpflasters vorliegt.

3. Das transdermale Absorptionspräparat gemäß Anspruch 1, wobei das Präparat ein homogenes Flüssigpräparat ist, das im Wesentlichen die Komponenten (a) bis (d) enthält.

4. Das transdermale Absorptionspräparat gemäß Anspruch 1, wobei das Präparat eine ölige Salbe oder ölige Creme ist, die im Wesentlichen die Komponenten (a) bis (d) und eine ölige Grundlage enthält.

5. Das transdermale Absorptionspräparat gemäß Anspruch 1, wobei die Komponenten (b) und (c) gegenseitig löslich mit Komponente (a) sind.

6. Das transdermale Absorptionspräparat gemäß Anspruch 1, wobei das Verhältnis des Gesamtgewichts der Komponenten (b) und (c) zum Gewicht der Komponente (a) 0,01-99 und das Gewichtsverhältnis der Komponente (c) zu Komponente (b) 0,1-10 beträgt.

## Revendications

1. Préparation pour absorption par voie transdermique comprenant les composants (a), (b), (c) et (d) suivants :
(a) du propylèneglycol,
(b) un ester d'acide gras et de polyol,
(c) du lauromacrogol, et
(d) du chlorhydrate de lopéramide ou de la lidocaïne en tant que composant médicamenteux.

2. Préparation pour absorption par voie transdermique selon la revendication 1, laquelle préparation est sous la forme d'une pommade, d'un gel, d'une crème, d'une crème analogue à un gel, d'un liquide, d'une lotion, d'un aérosol, d'un liniment, d'un emplâtre, d'un cataplasme, ou d'un timbre du type à réservoir.

3. Préparation pour absorption par voie transdermique selon la revendication 1, laquelle préparation est une préparation liquide homogène comprenant fondamentalement les composants (a) à (d).

4. Préparation pour absorption par voie transdermique selon la revendication 1, laquelle préparation est une préparation en pommade grasse ou en crème grasse comprenant les composants (a) à (d) et une base grasse.

5. Préparation pour absorption par voie transdermique selon la revendication 1, dans laquelle les composants (b) et (c) sont mutuellement solubles avec le composant (a).

6. Préparation pour absorption par voie transdermique selon la revendication 1, dans laquelle le rapport au poids total du composant (b) et du composant (c) sur le poids du composant (a) est de 0,01 à 99 et le rapport en poids du composant (c) sur le composant (b) est de 0,1 à 10.
